# FASCICULE DE BREVET EUROPEEN

(11) **EP 3 821 231 B1**
(45) Date de publication et mention de la délivrance du brevet: **10.09.2025**
(21) Numéro de dépôt: 19740539.2
(22) Date de dépôt: 12.07.2019
(51) Int. Cl.: G01N 21/3563, G01N 21/64, G01N 33/10, G01N 21/3554, G01N 21/359, G01N 21/17, G01N 21/85

(54) **DISPOSITIF D'ANALYSE DE GRAINS PAR SPECTROSCOPIE DE FLUORESCENCE ET INFRAROUGE**
VORRICHTUNG ZUR ANALYSE VON GETREIDE MITTELS FLUORESZENZ- UND INFRAROTSPEKTROSKOPIE
DEVICE FOR GRAIN ANALYSIS BY FLUORESCENCE AND INFRARED SPECTROSCOPY

(30) Priorité: 13.07.2018 FR 1856531
(43) Date de publication de la demande: 19.05.2021
(73) Titulaire: Spectralys Innovation, 92600 Asnières-sur-Seine (FR)
(72) Inventeur: VOISIN, Clément, 62690 Hermaville (FR); CHARLES-FRANCOIS, Olivier, 75011 Paris (FR); BOUTAOUAKOU, Papus, 77700 Chessy (FR); MESSAOUDI, Monji, 93300 Aubervilliers (FR); ODDOS, Stéphane, 92100 Boulogne Billancourt (FR)
(74) Mandataire: Gevers & Orès
(86) Numéro de dépôt international: PCT/EP2019/068916
(87) Numéro de publication internationale: WO 2020/012029

(56) Documents cités:
- EP-A1- 1 850 117
- EP-A2- 2 148 185
- WO-A1-2017/134050
- DE-A1- 10 119 763

## Description

L'invention se rapporte au domaine de l'analyse spectroscopique. En particulier, l'invention concerne un dispositif de mesure des propriétés d'un échantillon par spectroscopie de fluorescence et par spectroscopie infrarouge.

### Arrière-plan de l'invention

L'invention peut être appliquée en particulier, mais pas uniquement, à l'industrie pharmaceutique, à l'industrie environnementale ou encore à l'industrie agroalimentaire. Dans l'industrie agroalimentaire, les procédures industrielles nécessitent une connaissance précise des propriétés technologiques, nutritionnelles et/ou toxicologiques des échantillons analysés. Dans ce cadre, l'analyse d'échantillons au moyen de techniques spectroscopiques permet d'en extraire des paramètres avec un haut contenu informationnel physico-chimique.

Pour mesurer ces paramètres avec précision et en conformité avec les normes industrielles, il est connu des dispositifs de spectroscopie ayant recours à différentes méthodes. En particulier dans le cas d'échantillons de grains, la spectroscopie de fluorescence et la spectroscopie infrarouge permettent de mesurer séparément différents paramètres de ces échantillons. Le document EP 2 148 185 A2 décrit un dispositif d'analyse spectroscopique d'un échantillon de grains comprenant un module d'analyse infrarouge de l'échantillon et un module d'analyse du poids spécifique de l'échantillon. Les documents WO 2017/134050 A1 et EP 1 850 117 A1 divulguent des dispositifs d'analyse spectroscopique de grains comprenant un module pour acquérir des spectres infrarouges de l'échantillon, un module d'analyse de fluorescence de l'échantillon et un module de traitement configuré pour coupler les données acquises par lesdites modules et pour déterminer un indicateur de qualité du grain à partir des données couplées.

Cependant, un inconvénient des dispositifs connus est qu'ils ne sont conçus que pour fonctionner dans de petits espaces, par exemple dans des laboratoires de silos ou d'usine, avec un temps d'analyse très réduit, soit environ 1 à 3 minutes par échantillon. Or, la mesure doit être réalisée deux fois : d'une part au moyen d'un dispositif spectroscopique de fluorescence et d'autre part au moyen d'un dispositif spectroscopique infrarouge. De tels dispositifs séparés impliquent donc un temps, un espace et un coût d'acquisition plus élevés, une gestion des données plus complexe car chaque instrument est contrôlé par des moyens de traitement et des logiciels différents, et une logistique de surveillance des dispositifs et des sites de mesures plus lourde. En outre, la fiabilité et la cohérence des mesures réalisées au moyen de dispositifs séparés est significativement réduite étant donné que ces mesures sont réalisées sur des échantillons différents.

Par ailleurs, un autre inconvénient est que les dispositifs connus compliquent significativement le traitement conjoint des mesures obtenues de plusieurs technologies spectroscopiques différentes. Ainsi, les dispositifs connus sont souvent limités à déterminer les paramètres d'un échantillon de manière séparée et ne sont pas adaptés pour les mesurer de manière combinée, par exemple via un traitement conjoint de mesures de spectroscopie de fluorescence dans les domaines de l'ultraviolet et de mesures de spectroscopie infrarouge.

Dans les dispositifs connus, la mesure et le traitement des données issues de ces deux types de spectroscopies s'effectuent au sein de dispositifs séparés et non couplés, ce qui limite fortement leur utilisation conjointe et donc les synergies résultantes dans l'analyse des données. L'absence de convergence technique entre les deux modes d'acquisition rend également la tâche plus lourde et complexe.

### Objet et résumé de l'invention

L'invention vise à pallier l'un au moins des inconvénients précités.

Dans ce but, la présente invention propose un dispositif unique apte à réaliser une mesure de spectres de fluorescence, de spectres infrarouge et de poids spécifique sur un même échantillon.

Un aspect de l'invention concerne ainsi un dispositif d'analyse spectroscopique d'un échantillon de grains, ledit dispositif comprenant un premier module d'analyse infrarouge, un deuxième module d'analyse de fluorescence, un troisième module d'analyse de poids spécifique, un module de traitement et un réseau de communication, chacun desdits premier module et deuxième module comprenant
- une chambre de mesure configurée pour accueillir au moins une partie de l'échantillon ;
- un sous-module d'excitation configuré pour émettre au moins un rayonnement électromagnétique vers ladite au moins une partie de l'échantillon ;
- un sous-module de mesure configuré pour acquérir au moins un spectre électromagnétique de l'échantillon ;
- un système de vidange configuré pour guider l'échantillon vers ledit troisième module ;
le troisième module d'analyse comprenant
- un récipient configuré pour accueillir l'échantillon ;
- un sous-module de mesure configuré pour mesurer un poids spécifique de l'échantillon ;
ledit module de traitement étant connecté à chacun des modules d'analyse par le réseau de communication et comprenant
- une mémoire configurée pour recevoir des données transmises par ledit réseau de communication, lesdites données comprenant des spectres électromagnétiques acquis et des poids spécifiques mesurés ; et
- un processeur configuré pour organiser et coupler les données reçues dans la mémoire et pour déterminer un indicateur de qualité de l'échantillon à partir des données couplées.

Selon différentes caractéristiques supplémentaires dudit dispositif qui pourront être prises ensembles ou de façon séparée :
- le sous-module d'excitation du premier module d'analyse infrarouge est configuré pour émettre au moins un rayonnement électromagnétique de longueur d'onde comprise entre 600 et 2500 nanomètres, et dans lequel le sous-module de mesure du premier module d'analyse infrarouge comprend un spectromètre configuré pour acquérir au moins un spectre d'absorbance et/ou de transmittance ;
- le sous-module d'excitation du deuxième module d'analyse de fluorescence est configuré pour émettre au moins un rayonnement électromagnétique de longueur d'onde comprise entre 200 et 800 nanomètres et le sous-module du deuxième module d'analyse de fluorescence comprend un spectromètre configuré pour acquérir au moins un spectre sélectionné parmi : un spectre de fluorescence en mode frontal, acquis à un angle compris entre 30 et 60° par rapport à la surface de l'échantillon, soit un spectre de fluorescence classique, acquis à angle droit, une longueur d'onde dudit au moins un spectre étant comprise entre 200 nanomètres et 800 nanomètres ;

- ledit dispositif comprend en outre une trémie configurée pour guider au moins une partie de l'échantillon vers le premier module et le deuxième module ;
- ledit indicateur de qualité de l'échantillon est choisi parmi : un indice de chute de Hagberg, un taux de contamination en mycotoxines, un taux de contamination en acrylamide, un taux d'humidité, un taux de protéines, une teneur en sucres, une dureté, une force boulangère ou autre caractéristique typique des farines, une granulométrie ou encore un poids spécifique ;
- la hauteur du dispositif est inférieure à 75 centimètres, de préférence inférieure à 60 centimètres, la largeur du dispositif est inférieure à 70 centimètres, de préférence inférieure à 55 centimètres, et la profondeur du dispositif est inférieure à 55 centimètres, de préférence inférieure à 50 centimètres.

Avantageusement, le dispositif permet une mesure successive et/ou simultanée de données spectrales et de données de poids spécifique d'un échantillon, ce qui apporte une synergie d'informations améliorant la performance de la prédiction d'indicateurs de qualité habituellement mesurés par une seule des technologies infrarouge ou de fluorescence.

Avantageusement, la présence d'un sous-module de mesure configuré pour mesurer un poids spécifique d'un échantillon fournit un moyen de pesée qui permet de déterminer un critère majeur de la qualité des grains, par exemple en vue de déterminer le prix et l'usage ultérieur de ces grains.

Avantageusement, le dispositif permet en outre d'assurer une qualité spectrale homogène sur un spectre plus large, ce qui facilite la mise en commun des spectres pour leur exploitation ultérieure lors d'une fusion.

Avantageusement, le dispositif est miniaturisable et physiquement intégrable dans un système comprenant plusieurs modules d'analyse avec un volume et un poids réduits.

Avantageusement, le dispositif est apte à traiter un grand volume d'échantillon, ce qui est particulièrement intéressant lorsque les grains sont très hétérogènes, afin de réduire l'impact de l'échantillonnage sur la qualité de la calibration et de la prédiction.

Avantageusement, le temps d'analyse avant calibration est réduit de deux fois, puisque une seule analyse génère les deux sorties spectrales.

Avantageusement, le coût du dispositif est réduit ce qui permet de baisser très significativement le prix de cession de celui-ci par rapport à deux voire trois dispositifs distincts mettant en œuvre séparément une mesure spectroscopique infrarouge, une mesure spectroscopique de fluorescence et une mesure de poids spécifique.

Dans la suite de la description, on comprendra que le poids spécifique d'un échantillon est le poids par unité de volume de cet échantillon, et mesuré en kilogramme par hectolitre, par exemple de grains. On notera que la définition du poids spécifique est propre à la mesure sur grains entiers et répond aux normes en vigueur : le poids spécifique n'est pas exactement égal au poids volumique d'un échantillon tel qu'un fluide ou un solide.

### Brève description des dessins

D'autres caractéristiques et avantages de la présente invention ressortiront de la description faite ci-dessous, en référence aux dessins annexés qui en illustrent des exemples de réalisation dépourvus de tout caractère limitatif.

Sur les figures :
- les **figures 1a** et **1b** représentent, respectivement, une vue de côté et une vue du dessus d'une trémie de distribution d'un dispositif selon un mode de réalisation de l'invention ;
- les **figures 2a, 2b** et **2c** représentent, respectivement, une vue en perspective, une vue de côté et une vue du dessus d'un premier module d'analyse d'un dispositif selon un mode de réalisation de l'invention ;
- les **figures 3a** et **3b** représentent, respectivement, une vue en perspective et une vue du dessus d'un deuxième module d'analyse d'un dispositif selon un mode de réalisation de l'invention ;
- les **figures 4a, 4b** et **4c** représentent, respectivement, une vue en perspective, une vue de côté et une vue du dessus d'un troisième module d'analyse d'un dispositif selon un mode de réalisation de l'invention ;
- la **figure 5** représente une vue en perspective d'un dispositif selon un mode de réalisation de l'invention ;
- la **figure 6** représente une vue de côté d'un dispositif selon un mode de réalisation de l'invention ; et
- la **figure 7** représente, sous forme d'organigramme, des étapes d'un procédé d'analyse selon un mode de réalisation de l'invention.

Naturellement, pour satisfaire des besoins spécifiques, une personne compétente dans le domaine de la technique pourra appliquer des modifications dans la description suivante. Bien qu'elle se réfère à différents modes de réalisation, la présente invention n'est pas limitée à ces modes de réalisation spécifiques, et toutes modifications propres au champ d'application de la présente invention peuvent être considérées comme évidentes pour une personne versée dans l'art de la technique correspondant.

### Description détaillée d'un mode de réalisation

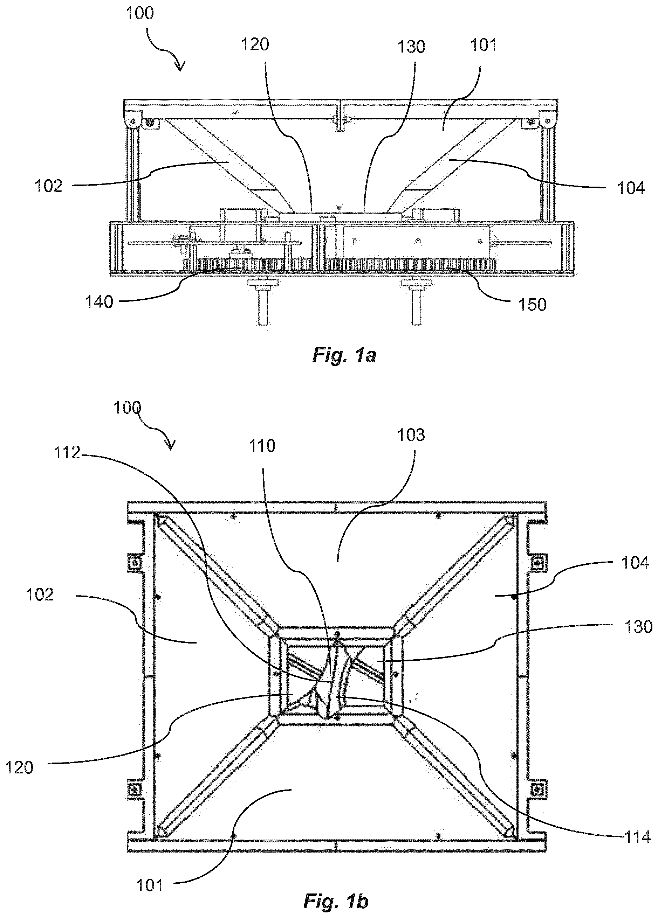

Les figures 1a et 1b montrent une vue de côté et une vue du dessus d'une trémie 100 de distribution d'un dispositif 1 selon l'invention. Un échantillon, par exemple un échantillon de grains, est versé au-dessus de la trémie 100 qui guide celui-ci par la suite vers d'autres éléments du dispositif 1, comme décrit ci-après.

De manière non limitative, il sera considéré dans la suite de la description que l'échantillon versé dans la trémie 100 est un échantillon de grains. En toute généralité, cet échantillon peut aussi comprendre tout type de graines, de farines et/ou de semoules, par exemple de l'orge, du blé, du malt, du maïs, du seigle, du colza, de l'avoine, du triticale, du soja, du tournesol, du sarrasin, de l'épeautre, des pois, de la féverole, des lentilles, des vesces et/ou du chènevis.

Selon un mode de réalisation de l'invention, la trémie 100 présente la forme d'un entonnoir, par exemple un entonnoir rectangulaire comportant quatre faces 101, 102, 103 et 104. Ces quatre faces sont inclinées et adaptées pour guider l'échantillon versé vers la partie inférieure de la trémie 100 sous l'effet de la gravité. Chacune des faces 101, 102, 103 et 104 est reliée à au moins une sortie parmi une première sortie 120 et une deuxième sortie 130, chacune de ces deux sorties étant située dans la partie inférieure de la trémie 100.

Selon un mode de réalisation de l'invention, les deux sorties 120 et 130 de la trémie 100 sont séparées l'une de l'autre par au moins une paroi 110, par exemple une paroi verticale, comprenant deux faces 112 et 114. Les deux faces 112 et 114 sont disposée(s) de sorte à séparer et à distribuer l'échantillon s'écoulant dans la trémie 100 en deux volumes, un premier volume étant guidé hors de la trémie 100 via la première sortie 120 et un deuxième volume étant guidé hors de la trémie 100 via la deuxième sortie 130. En général, le premier volume et le deuxième volume sont différents.

Les faces de la trémie 100 peuvent être réalisées en un seul type de matériau ou en différents types de matériaux, comprenant par exemple du métal, de l'inox ou du plastique, et sont conçues pour permettre un écoulement fluide, continu et sans perte de l'échantillon versé depuis la partie supérieure de la trémie vers l'une des deux sorties 120 et 130.

Les deux sorties 120 et 130 de la trémie 100 sont reliées à au moins un module d'analyse au moyen d'un guide d'acheminement, par exemple un tuyau, une glissière ou un tapis roulant, qui est configuré pour guider l'échantillon vers ce ou ces modules d'analyse. Pour améliorer le guidage de l'échantillon, des roues segmentées 140 et 150 peuvent être disposées entre les sorties de la trémie et le guide d'acheminement pour faire circuler les grains depuis la trémie vers le guide d'acheminement.

Selon un mode de réalisation de l'invention, la première sortie 120 et la deuxième sortie 130 sont des roues de distribution. De préférence, la première sortie 120 est reliée à un premier module 200 d'analyse, et la deuxième sortie 130 est reliée à un deuxième module 300 d'analyse. En variante, la première sortie 120 et la deuxième sortie 130 sont reliées en outre à un troisième module 400 d'analyse.

Selon d'autres modes de réalisation non représentés de la présente invention, la trémie 100 présente la forme d'un entonnoir circulaire. De manière non limitative, la trémie 100 comporte un nombre quelconque de faces, lesdites faces pouvant présenter des formes diverses, et qui forment un entonnoir conique, cylindrique ou carré. Les deux sorties 110 et 120 ainsi que la paroi 130 peuvent être adaptées à ces différents exemples.

En référence à la figure 1b, un échantillon de grains versé au-dessus de la trémie 100 est guidé vers la première sortie 120 lorsque celui-ci s'écoule le long de la face 102 ou le long d'une partie de la face 103 et vers la deuxième sortie 130 lorsqu'il s'écoule le long de la face 104, le long d'une partie de la face 103 ou le long d'une partie de la face 101. Selon un autre exemple non représenté, un échantillon de grains versé au-dessus de la trémie 100 est guidé vers la première sortie 120 lorsque celui-ci s'écoule le long de la face 102 et le long de la face 103 de la trémie, et vers la deuxième sortie 130 lorsqu'il s'écoule le long de la face 104 et le long de la face 101.

Selon un mode de réalisation de l'invention, la paroi 110 peut être déplacée et/ou orientée en vue de modifier la manière dont est séparé l'échantillon en deux volumes, et donc pour définir des valeurs spécifiques du premier volume guidé hors de la trémie 100 via la première sortie 120 et du deuxième volume guidé hors de la trémie 100 via la deuxième sortie 130. Par exemple, la paroi 110 peut être déplacée et/ou orientée par un dispositif de contrôle afin de modifier la disposition ou la taille relative de la première sortie 120 et de la deuxième sortie 130.

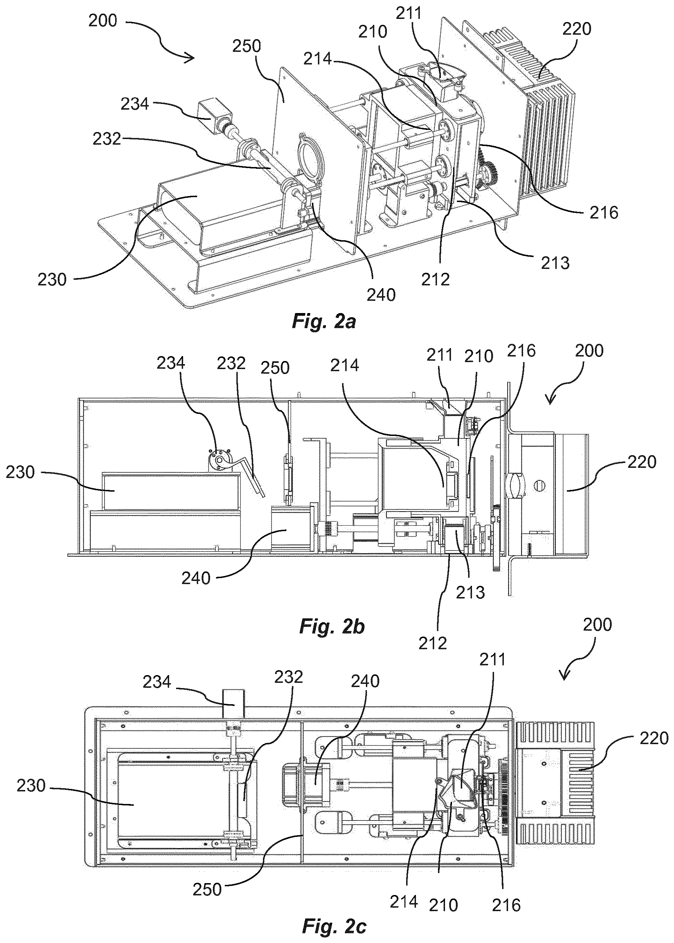

Les figures 2a, 2b et 2c montrent une vue en perspective, une vue de côté et une vue du dessus d'un premier module 200 d'analyse d'un dispositif 1 selon un mode de réalisation de la présente invention.

En particulier, le premier module 200 d'analyse est un module d'analyse infrarouge configuré pour acquérir un ou plusieurs spectres d'un échantillon dans le proche infrarouge, par exemple un ou plusieurs spectres d'absorbance et/ou de transmittance. Ledit module d'analyse infrarouge peut également être configuré pour acquérir un ou plusieurs spectres de réflectance.

Dans le cadre des principes de la spectroscopie infrarouge, le spectre infrarouge ou proche infrarouge d'un échantillon est établi en faisant passer un faisceau électromagnétique de longueur d'onde comprise entre 600 et 2500 nanomètres au travers de cet échantillon. Selon un mode de réalisation de l'invention, le faisceau électromagnétique est émis par une source large continue à large bande. En variante, d'autres types de sources peuvent être utilisées, par exemple une ou plusieurs sources monochromatiques.

L'échantillon soumis à une ou plusieurs sources de longueurs d'onde infrarouge émet alors un spectre de rayonnements électromagnétiques. L'analyse de ces rayonnements électromagnétiques et de la quantité d'énergie correspondante permet d'en en déduire les spectres en absorbance et/ou en transmittance de l'échantillon et, par la suite, de mesurer des paramètres tels que le taux d'humidité, le taux de protéines, la teneur en sucres, la dureté ou encore la granulométrie d'un échantillon.

Le premier module 200 d'analyse comprend une première chambre 210 de mesure configurée pour accueillir et contenir un échantillon. Selon un mode de réalisation de l'invention, la première chambre 210 comprend une entrée 211 pour accueillir cet échantillon, en particulier le premier volume de l'échantillon guidé hors de la trémie 100. De préférence, l'entrée 211 comprend un entonnoir adapté pour guider l'échantillon vers l'intérieur de la première chambre 210 et pour remplir celle-ci sans pertes, de manière manuelle ou automatique.

Selon un mode de réalisation de l'invention, la première chambre 210 comporte des éléments de détection, par exemple des détecteurs optiques, mécaniques ou électroniques, configurés pour déterminer et indiquer si un échantillon est présent dans la première chambre 210, et de préférence quel volume de cet échantillon. Ces éléments de détection comprennent, par exemple, un capteur de détection combinant un émetteur infrarouge et une photodiode pour permettre une détection de la présence d'un échantillon dans la première chambre 210, et en déduire si celle-ci est remplie totalement, partiellement ou pas du tout.

Selon un mode de réalisation de l'invention, la première chambre 210 comprend en une première extrémité une première paroi 214 et en une deuxième extrémité une deuxième paroi 216.

Selon un mode de réalisation de l'invention, une partie de la paroi 214 et de la paroi 216 comprend une fenêtre transparente, par exemple une fenêtre en verre ou en plastique. Au moins l'une des deux parois 214 et 216 est partiellement ou complètement transparente aux rayonnements électromagnétiques. De préférence, la fenêtre de la paroi 216 est dépolie de manière à être partiellement ou complètement transparente aux rayonnements électromagnétiques infrarouges. Avantageusement, l'utilisation d'au moins une fenêtre ainsi dépolie améliore la qualité des spectres mesurables de l'échantillon, et limite la gamme dynamique entre ceux-ci et des spectres de référence mesurés en l'absence de l'échantillon.

Sur les figures 2a, 2b et 2c, la paroi 216 est disposée du côté d'un premier sous-module 220 d'excitation et la paroi 214 est disposée du côté du sous-module 230 de mesure. De manière équivalente, le premier sous-module 220 d'excitation est un sous-module d'illumination.

La première chambre 210 comprend une troisième sortie 212 pour évacuer et guider l'échantillon hors de celle-ci et hors du premier module d'analyse 200 vers un autre élément du dispositif 1, ou hors du dispositif 1. Par exemple, la troisième sortie 212 peut guider l'échantillon vers une entrée du deuxième module d'analyse 300 ou du troisième module 400 d'analyse. La troisième sortie 212, qui peut être soit en position ouverte ou en position fermée, est commandée manuellement ou automatiquement.

Selon un mode de réalisation de l'invention, une commande de la troisième sortie 212 du premier module 200 d'analyse est réalisée au moyen d'un premier système 213 de vidange, ce premier système 213 de vidange comportant par exemple un moteur qui peut être commandé pour ouvrir et fermer automatiquement la troisième sortie 212.

Selon un mode de réalisation de l'invention, la première paroi 214 est une paroi mobile et la deuxième paroi 216 est une paroi fixe. En particulier, la paroi 214 est déplaçable le long de l'axe principal du premier module 200 d'analyse au moyen d'un moteur 240. Le déplacement de la paroi 214 par rapport à la paroi 216 permet d'augmenter ou de diminuer la taille de la première chambre 210, et ainsi de modifier l'épaisseur ou le volume maximal d'un échantillon pouvant être contenu dans la première chambre 210. Le déplacement de la paroi 214 par rapport à la paroi 216, qui peut être automatisé, permet de positionner celle-ci avec une précision inférieure à 0,05 millimètre en quelques secondes.

En raison de la grande diversité des échantillons analysables par le présent dispositif, par exemple de par la taille des grains qui composent cet échantillon, la densité volumique de l'échantillon contenu dans la première chambre 210 et les propriétés de diffusion optique de cet échantillon peuvent varier fortement d'un cas à un autre. Par exemple, les propriétés de diffusion, d'absorption et d'amplification spectrale d'échantillons de blé, de maïs et d'orge peuvent être très différentes. Avantageusement, un ajustement de la taille de la première chambre 210 en fonction de l'échantillon présent permet d'optimiser la qualité des mesures effectuées par le premier module 200 d'analyse lorsque celui-ci est un module d'analyse infrarouge configuré pour acquérir un ou plusieurs spectres d'absorption ou de transmission d'un échantillon dans l'infrarouge ou dans le proche infrarouge.

Le premier module 200 d'analyse comprend en outre un premier sous-module 220 d'excitation et un deuxième sous-module de mesure 230. Selon un mode de réalisation de l'invention, et lorsque le premier module 200 d'analyse infrarouge est configuré pour acquérir un ou plusieurs spectres de transmittance, chacun de ces deux sous-modules étant disposé de part et d'autre de la première chambre 210. En variante, ces deux sous-modules peuvent être placés d'un même côté de la première chambre 210, par exemple pour permettre l'acquisition de spectres de réflectance.

Le premier sous-module 220 d'excitation est configuré pour émettre un rayonnement électromagnétique, en particulier un rayonnement électromagnétique dont la longueur d'onde est dans l'infrarouge. Ce rayonnement est émis par le premier sous-module 220 d'excitation en direction de la première chambre 210 et du deuxième sous-module 230 de mesure. Le premier sous-module 220, la première chambre 210 et le deuxième sous-module 230 sont alignés le long d'un axe définissant sensiblement la trajectoire des rayonnements émis par le premier sous-module 220 d'excitation, puis traversant la première chambre 210 et reçus par le deuxième sous-module 230 de mesure.

Selon un mode de réalisation de l'invention, le premier sous-module 220 d'excitation comprend une source de lumière continue à large bande, ladite source de lumière étant configurée pour émettre un rayonnement électromagnétique dont la longueur d'onde est comprise entre 600 et 2500 nanomètres.

Par exemple, le sous-module d'excitation 220 comprend une lampe quartz-tungstène-halogène (QTH), qui émet un rayonnement lumineux à partir d'un filament de tungstène chauffé. De préférence, ladite lampe quartz-tungstène-halogène est configurée pour être sous-alimentée avec une puissance comprise entre 25 Watts et 50 Watts, et pour émettre un rayonnement dans une gamme de longueurs d'onde comprises entre 240 et 2700 nanomètres.

Le premier sous-module 220 d'excitation est de préférence disposé de sorte à ce que les rayonnements sont émis en direction de l'échantillon. Ces rayonnements sont ensuite absorbés par l'échantillon, ou diffusés par l'échantillon et détectés par le deuxième sous-module 230 de mesure, comme détaillé ci-après. Le deuxième sous-module 230 de mesure est configuré pour transmettre les mesures effectuées à tout moment vers un module de traitement.

La spectroscopie d'absorption repose sur le principe selon lequel tout matériau soumis à un rayonnement incident, par exemple un rayonnement infrarouge, peut soit réfléchir une partie de ces rayonnements, soit absorber une partie de ces rayonnements, soit transmettre une partie de ces rayonnements. En particulier, l'absorption des rayonnements par l'échantillon amène celui-ci à générer un ou plusieurs spectre(s) d'absorption et/ou de transmission.

Le deuxième sous-module 230 de mesure est disposé et configuré pour recevoir des rayonnements électromagnétiques émis par le premier sous-module 220 d'excitation et des rayonnements électromagnétiques provenant de la première chambre 210 et/ou d'un échantillon contenu dans cette première chambre 210. Selon un mode de réalisation de l'invention, le deuxième sous-module 230 de mesure comprend un spectromètre haute sensibilité qui est configuré pour acquérir un ou plusieurs spectres, par exemple dans le proche infrarouge.

Selon un mode de réalisation de l'invention, un spectromètre du deuxième sous-module 230 de mesure est intégré dans une technologie CMOS, par exemple sur une plaquette de silicium sur isolant (SOI). Selon un autre mode de réalisation de l'invention, le spectromètre du deuxième sous-module 230 de mesure comporte un capteur de type CCD, par exemple une barrette de détection BI-CCD avec une fente de l'ordre de 200 micromètres. Avantageusement, un tel spectromètre est sensible aux rayonnements électromagnétiques infrarouges émis par une large gamme d'échantillons, en particulier dans la gamme des longueurs d'onde comprises entre 850 et 1100 nanomètres.

La gamme dynamique d'un spectromètre définit sa gamme de détection, correspondant au rapport de l'intensité du signal le plus grand et de l'intensité du signal le plus petit mesurables par ce spectromètre. En particulier, un spectromètre du deuxième sous-module 230 de mesure est caractérisé par une gamme dynamique égale au niveau maximal divisé par le niveau minimal d'un signal mesurable par ce spectromètre, la mesure d'un signal de niveau minimal correspondant à une mesure réalisée en l'absence d'un signal. Généralement, une mesure réalisée en l'absence d'un signal peut être mise en œuvre à l'aide de d'une ou de plusieurs acquisitions de spectres en l'absence de signal, par exemple 25 à 50 acquisitions, et en calculant la moyenne, la valeur efficace et/ou la valeur quadratique de ces acquisitions pour définir la gamme dynamique du spectromètre.

Selon un mode de réalisation de l'invention, les éléments du premier module 200 d'analyse sont disposés de manière fixe pour éviter tout mouvement de pièce optique, à l'exception de la paroi 214 si celle-ci est fixe. En outre, un spectromètre du deuxième sous-module 230 de mesure peut être fixé à celui-ci par un ou plusieurs supports mécaniques. Avantageusement, une telle configuration fournit une chaine optique qui permet une acquisition stable et précise de spectres de transmittance, en particulier dans le proche infrarouge.

Selon un mode de réalisation de l'invention, la première chambre 210 comporte des éléments diffuseurs. Ces éléments diffuseurs peuvent être placés en entrée de la première chambre 210 à proximité de la paroi 216 et du premier sous-module 220 d'excitation et/ou à proximité de la paroi 214 et du deuxième sous-module 230 de mesure. Lorsqu'un échantillon est présent dans la première chambre 210, une grande partie du rayonnement électromagnétique émis par le premier sous-module 220 d'excitation est absorbé par celui-ci et une partie moindre de ce rayonnement est transmis en direction du deuxième sous-module 230 de mesure. En général, un échantillon illuminé par un rayonnement infrarouge absorbe une fraction importante de ce rayonnement, ce qui limite l'intensité du signal mesuré par un spectromètre.

Avantageusement, le placement d'éléments diffuseurs, et en particulier de diffuseurs infrarouge, en entrée et/ou en sortie de la première chambre 210 permet d'améliorer la qualité des spectres de transmittance mesurés par le deuxième sous-module 230 de mesure dans le domaine de l'infrarouge et du proche infrarouge. En particulier, le placement d'éléments diffuseurs réduit la gamme dynamique à détecter d'au moins un ordre de grandeur sans modifier significativement le temps nécessaire à l'acquisition des spectres, de l'ordre de 1 % du temps de mesure total.

Selon un mode de réalisation de l'invention, les éléments diffuseurs comprennent des surfaces de diffusion réalisées en un ou plusieurs matériaux choisis parmi du silicium, du silice, du saphir ou de tout type de matériau permettant une transmission élevée des rayonnements électromagnétiques dans les gammes de longueurs d'onde de l'infrarouge et du proche infrarouge. Par exemple, des éléments diffuseurs réalisés en un matériau de silicium transmettent de préférence les rayonnements infrarouge, mais pas les rayonnements de lumière visible. Avantageusement, la granularité des éléments diffuseurs peut être variée et sélectionnée en vue de combiner plusieurs d'entre eux pour obtenir le meilleur compromis entre réduction de la gamme dynamique et limitation de la perte du signal mesuré.

Pour mesurer un signal et/ou un spectre de niveau minimal, le spectromètre du deuxième sous-module 230 de mesure comprend un obturateur 232, par exemple une plaque ou un élément opaque, qui est connecté à un élément 234 de rotation, par exemple un moteur, pour déplacer l'obturateur. L'obturateur 232 est déplaçable manuellement ou automatiquement entre une position éloignée de l'entrée du spectromètre et une position située devant l'entrée de ce spectromètre. Lorsque l'obturateur 232 est en une position éloignée, le spectromètre n'est pas occulté et reçoit tous les rayonnements électromagnétiques émis vers lui ; lorsque l'obturateur 232 est devant l'entrée du spectromètre, le spectromètre est occulté et aucun rayonnement électromagnétique n'est mesurable par celui-ci. Ce mécanisme permet au spectromètre d'acquérir un spectre issu d'un échantillon contenu dans la première chambre 210 lorsque le spectromètre n'est pas occulté et un spectre de niveau minimal, ou de bruit, lorsque le spectromètre est occulté.

Selon un mode de réalisation de l'invention, le premier module 200 d'analyse comprend en outre une plaque 250 pour séparer et isoler le deuxième sous-module 230 de mesure de la première chambre 210. La plaque 250 comprend une ouverture qui laisse passer les rayonnements électromagnétiques. Selon un autre mode de réalisation de l'invention (non représenté), aucune plaque de séparation n'est présente entre le deuxième sous-module 230 de mesure et la première chambre 210.

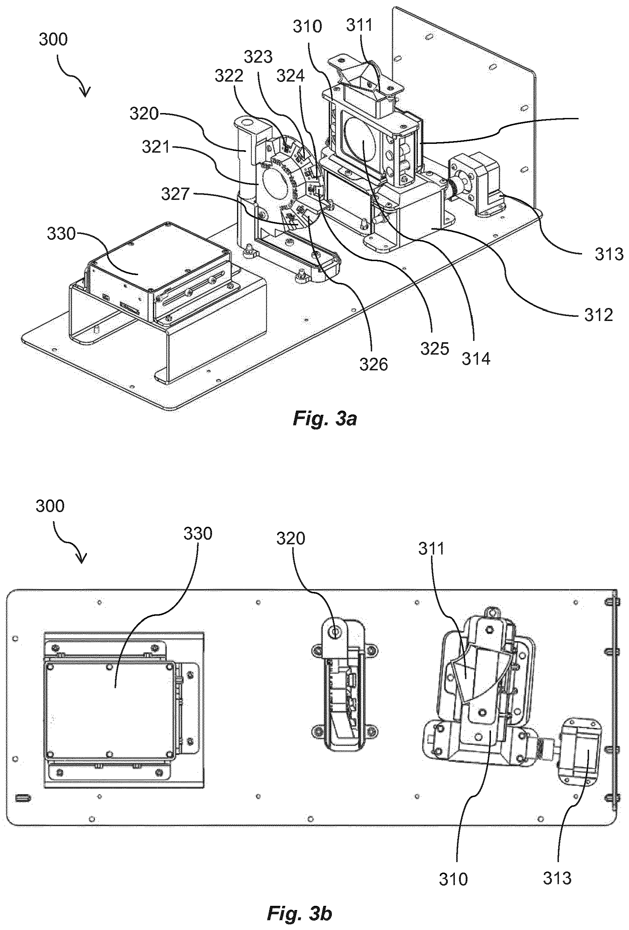

Les figures 3a et 3b montrent une vue en perspective et une vue du dessus d'un deuxième module 300 d'analyse d'un dispositif 1 selon un mode de réalisation de la présente invention.

En particulier, le deuxième module 300 d'analyse est un module d'analyse de fluorescence configuré pour acquérir un ou plusieurs spectres de fluorescence d'un échantillon, par exemple un ou plusieurs spectres de fluorescence frontale.

Dans le cadre des principes de la spectroscopie de fluorescence, un échantillon est excité par un rayonnement lumineux d'une longueur d'onde déterminée, par exemple dans le domaine du visible ou de l'ultraviolet. Les spectres de fluorescence s'étendent préférentiellement sur une plage spectrale comprise entre 200 nanomètres et 800 nanomètres. L'utilisation de ces longueurs d'onde dans le cadre de méthodes de spectroscopie de fluorescence classique ou frontale permet de mesurer des paramètres tels que l'indice de chute de Hagberg ou le taux de contamination en mycotoxines, acrylamide, etc. L'indice de chute de Hagberg, permet de mesurer l'activité de l'alpha-amylase présente dans des grains de blé, notamment, et/ou de détecter rapidement les échantillons contaminés ou endommagés, ou encore la pureté variétale, à leur entrée en silo.

En réponse à cette excitation, l'échantillon émet un rayonnement dont les propriétés dépendent des composants contenus dans cet échantillon. Sur base de la mesure de ce rayonnement d'émission, il est alors possible d'en déduire le ou les spectre(s) de fluorescence correspondant(s). Le traitement de ces spectres de fluorescence, de préférence acquis sur une plage spectrale comprise entre 200 nanomètres et 800 nanomètres, grâce à des outils de pré-traitement, de décomposition et de modélisation par apprentissage ou autre, permet d'extraire des informations telles que l'indice de chute de Hagberg, la contamination en mycotoxines ou d'autres paramètres caractérisant le degré de germination des graines contenues dans un volume de l'échantillon.

Le deuxième module 300 d'analyse comprend une deuxième chambre 310 de mesure configurée pour accueillir et contenir un échantillon. Selon un mode de réalisation de l'invention, la deuxième chambre 310 comprend une entrée 311 pour accueillir cet échantillon, par exemple le deuxième volume de l'échantillon guidé hors de la trémie 100. De préférence, l'entrée 311 comprend un entonnoir adapté pour guider l'échantillon vers l'intérieur de la première chambre 310 pour remplir celle-ci sans perte de manière manuelle ou automatique.

La deuxième chambre 310 comprend une quatrième sortie 312 pour évacuer et guider l'échantillon hors du deuxième module d'analyse 300 vers un autre élément du dispositif 1, par exemple vers une entrée d'un troisième module 400 d'analyse. La quatrième sortie 312, qui peut être soit en position ouverte ou en position fermée, est commandée manuellement ou automatiquement. En particulier, l'ouverture et/ou la fermeture de la quatrième sortie 312 peut être réalisée manuellement ou automatiquement au moyen d'un deuxième système 313 de vidange, qui peut être motorisé.

Le deuxième module 300 d'analyse est relié au dispositif 1. En particulier, la quatrième sortie 312 est reliée à au moins un module d'analyse, au moyen d'un guide. De préférence, la quatrième sortie 312 est reliée à un troisième module 400 d'analyse.

Selon un mode de réalisation de l'invention, la deuxième chambre 310 comporte en outre des éléments de détection, par exemple des détecteurs optiques, mécaniques ou électroniques, pour déterminer si un échantillon est présent dans la deuxième chambre 310, et de préférence, quel volume de cet échantillon est présent.

Le deuxième module 300 d'analyse comprend en outre un troisième sous-module 320 d'excitation et un quatrième sous-module 330 de mesure, la configuration de chacun de ces deux sous-modules étant de préférence tel que ceux-ci soient disposés d'un même côté de la deuxième chambre 310 de mesure pour permettre une mesure de spectres de fluorescence frontale.

En variante, d'autres configurations sont envisageables pour permettre l'acquisition de spectres de fluorescence classique ou frontale. Par exemple, dans le cas d'une configuration prévue pour l'acquisition de spectres de fluorescence frontale, l'ouverture 314 de la deuxième chambre 310 est disposée du même côté que le troisième sous-module 320 d'excitation et que le quatrième sous-module 330 de mesure. En variante, le troisième sous-module 320 d'excitation et le quatrième sous-module 330 de mesure peuvent être placés d'un côté différent de la deuxième chambre 310 pour le cas d'autres configurations.

Le troisième sous-module 320 d'excitation est configuré pour générer et émettre au moins un rayonnement électromagnétique en direction de la deuxième chambre 310, en particulier un rayonnement électromagnétique visible ou ultraviolet d'une longueur d'onde comprise entre 200 et 800 nanomètres.

Le troisième sous-module 320 d'excitation est fixé de manière stable dans le deuxième module 300 d'analyse et comprend une pièce optomécanique 321. Cette pièce optomécanique 321 comprend une ou plusieurs sources de lumière configurée(s) pour émettre des rayonnements électromagnétiques à des longueurs d'onde d'éclairage prédéterminées. De préférence, la ou chacune des sources de lumière émet un rayonnement monochromatique à une longueur d'onde donnée en direction de l'échantillon contenu dans la deuxième chambre 320. En fonction du nombre de sources de lumière, ces rayonnements électromagnétiques peuvent échantillonner grossièrement (plusieurs dizaines de longueurs d'onde) ou finement (plusieurs centaines de longueurs d'onde) une plage spectrale, par exemple une plage spectrale couvrant les domaines du visible et de l'ultraviolet.

De manière non limitative, les sources de lumière de la pièce optomécanique 321 comprennent une source de rayonnement monochromatique ou une source de rayonnement polychromatique. Par exemple, ces sources de lumière comportent une diode électroluminescente ou une source laser. Le troisième sous-module d'excitation 320 et/ou la pièce optomécanique 321 peuvent comprendre d'autres éléments optiques tels que des éléments de focalisation ou des éléments de diffusion, par exemple des lentilles. Par exemple, la pièce optomécanique 321 comprend une lentille configurée pour focaliser ou diffuser les rayonnements électromagnétiques la traversant.

Selon un mode de réalisation de l'invention, la pièce optomécanique 321 comprend une ouverture configurée pour laisser passer les rayonnements électromagnétiques, en particulier les rayonnements électromagnétiques émis par l'échantillon situé dans la deuxième chambre 310, en direction du quatrième sous-module 330 de mesure.

Selon un mode de réalisation de l'invention, la pièce optomécanique 321 est de forme circulaire et comprend un nombre donné de sources de lumière, ce nombre étant compris entre un et vingt, et de préférence compris entre un et dix.

Sur les figures 3a et 3b, le troisième sous-module d'excitation 320 comprend six diodes électroluminescentes disposées le long de la circonférence de la pièce optomécanique 321 circulaire. Les six diodes électroluminescentes 322 à 327, comprennent quatre diodes 322, 323, 324 et 325 configurées pour émettre un rayonnement ultraviolet d'une longueur d'onde centrale égale à 275 +/- 5 nanomètres, une diode 326 configurée pour émettre un rayonnement ultraviolet d'une longueur d'onde centrale égale à 338 +/- 3 nanomètres, une diode 327 configurée pour émettre un rayonnement ultraviolet d'une longueur d'onde centrale égale à 385 +/- 3 nanomètres. De manière générale, chaque diode peut être configurée pour fournir une puissance comprise entre 5 milliwatts et 1 watt. Le troisième sous-module d'excitation 320 peut également comporter une septième diode configurée pour émettre un rayonnement ultraviolet d'une longueur d'onde centrale égale à 420 +/- 5 nanomètres.

Lorsqu'il atteint l'échantillon contenu dans la deuxième chambre 320, un rayonnement électromagnétique émis par l'une des sources de lumière de la pièce optomécanique 321 provoque une excitation de l'échantillon. Lorsque cet échantillon se désexcite, celui-ci émet un spectre complet de fluorescence dans toutes les directions, et notamment en direction du quatrième sous-module 330 de mesure.

Selon un mode de réalisation de l'invention, la deuxième chambre 310, le troisième sous-module d'excitation 320 et le quatrième sous-module 330 de mesure sont alignés le long d'un axe correspondant à l'axe de la trajectoire des rayonnements électromagnétiques émis par l'échantillon dans la deuxième chambre 320 en direction du quatrième sous-module 330 de mesure. Le quatrième sous-module 330 de mesure est placé de sorte à pouvoir recevoir les rayonnements électromagnétiques provenant de l'échantillon contenu dans la deuxième chambre 310 après l'excitation de cet échantillon. Le quatrième sous-module 330 de mesure est configuré pour transmettre les mesures effectuées à tout moment vers un module de traitement

Selon un mode de réalisation de l'invention, le quatrième sous-module 330 de mesure comprend un spectromètre configuré pour mesurer un ou plusieurs spectre(s) de fluorescence, par exemple un spectre de fluorescence frontale dans l'ultraviolet et le visible, et correspondant à des longueurs d'onde comprises entre 200 et 800 nanomètres. Le spectromètre du quatrième sous-module 330 de mesure est disposé sensiblement du même côté que le troisième sous-module d'excitation 320 pour permettre l'acquisition de spectres de fluorescence frontale. Comparativement aux autres types de spectres de fluorescence, l'acquisition de spectres de fluorescence frontale permet d'éviter de générer de trop grandes erreurs analytiques liées à l'échantillon, à sa préparation ou aux conditions externes comme la température ou la pression ; les résultats obtenus sont donc plus précis et déterminés plus rapidement.

Tout comme pour le spectromètre du deuxième sous- module 230 de mesure, le spectromètre du quatrième sous-module 330 de mesure est configuré pour acquérir en outre un signal et/ou un spectre de niveau minimal. Cette acquisition peut être mise en œuvre sans nécessiter d'obturateur, en réalisant une mesure lorsque la ou les sources de lumière du troisième sous-module d'excitation 320 sont éteintes.

Selon un mode de réalisation de l'invention, le spectromètre du quatrième sous-module 330 de mesure comporte un capteur de type CCD avec une fente de l'ordre de 500 micromètres et une résolution d'environ 10 nanomètres.

Selon d'autres modes de réalisation non représentés, le troisième sous-module 320 d'excitation peut être disposé en différentes positions et orientations dans le deuxième module 300 d'analyse. Par exemple, le troisième sous-module 320 d'excitation peut être disposé le long d'un axe sensiblement perpendiculaire à un autre axe passant par la deuxième chambre 310 et par le deuxième sous-module 330 de mesure, afin d'acquérir des spectres de fluorescence à angle droit.

Selon un mode de réalisation de l'invention, la deuxième chambre 310, le troisième sous-module 320 d'excitation et le quatrième sous-module 330 de mesure sont fixés dans le deuxième module 300 d'analyse par un ou plusieurs supports mécaniques. Cette configuration évite tout mouvement de pièce mécanique, et fournit une chaine optique permettant une acquisition stable et précise de spectres de fluorescence, en particulier des spectres de fluorescence frontale et ou d'autres types de spectres. Avantageusement, le deuxième module 300 d'analyse est compact et n'intègre pas de pièces optiques mobiles. Cette compacité et cette stabilité améliorent la sensibilité et la répétabilité des mesures lors de l'acquisition de spectres de fluorescence tout en facilitant la standardisation instrumentale.

Selon un mode de réalisation de l'invention, le remplissage de la deuxième chambre 310, l'excitation de l'échantillon par le sous-module d'excitation 320 et l'acquisition de spectres de fluorescence par le deuxième sous-module de mesure 330 sont automatisés mécaniquement et électroniquement.

Selon un mode de réalisation de l'invention, une commande de la quatrième sortie 312 du deuxième module 300 d'analyse est réalisée au moyen d'un deuxième système 313 de vidange, ce deuxième système 313 de vidange comportant par exemple un moteur qui peut être commandé pour ouvrir et fermer la quatrième sortie 312.

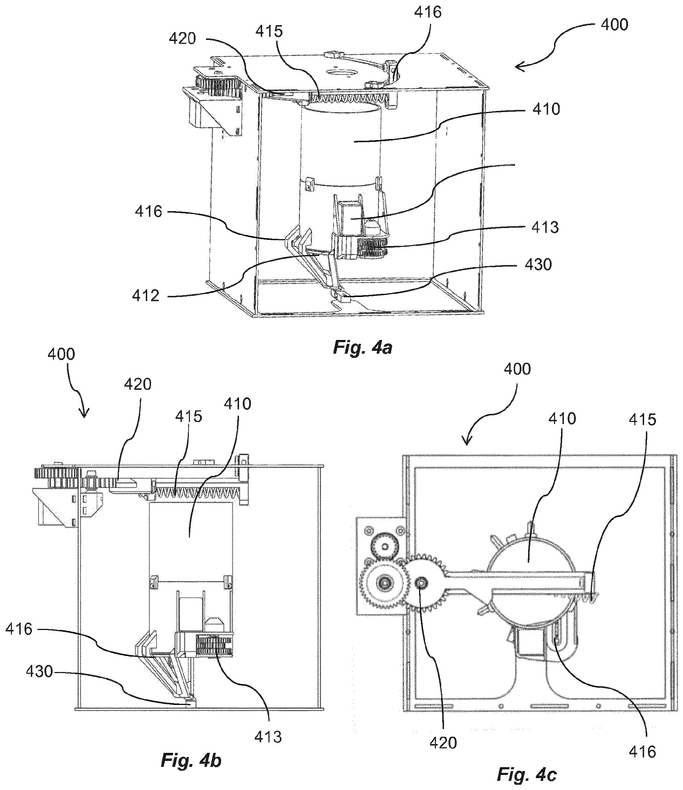

Les figures 4a, 4b et 4c montrent une vue en perspective, une vue de côté et une vue du dessus d'un troisième module d'analyse 400 d'un dispositif 1 selon un mode de réalisation de la présente invention. Selon un mode de réalisation de l'invention, le troisième module d'analyse 400 est un module de mesure de poids configuré pour mesurer le poids spécifique d'un échantillon

Le troisième module 400 d'analyse est connecté au reste du dispositif 1. Selon un mode de réalisation de l'invention, le troisième module d'analyse 400 est connecté à la troisième sortie 212 de la première chambre 210 et à la quatrième sortie 312 de la deuxième chambre 310 afin de mesurer le poids spécifique du même échantillon que celui issu du premier module 200 d'analyse et du deuxième module 300 d'analyse. En variante, le troisième module 400 d'analyse est connecté à la première sortie 120 et/ou à la deuxième sortie 130 de la trémie 100.

Le troisième module 400 d'analyse comprend un récipient 410 configuré pour accueillir et contenir une partie de l'échantillon. La base du récipient 410 est supportée par une plateforme 416 qui le stabilise et qui peut présenter différentes dimensions et géométries.

Selon un mode de réalisation de l'invention, le récipient 410 est un conteneur qui présente une forme cylindrique, parallélépipédique, ou encore conique. Ce conteneur comprend un volume de remplissage compris entre 100 millilitres et 1000 millilitres, de préférence 500 millilitres.

Selon un mode de réalisation de l'invention, le troisième module 400 d'analyse comprend un cinquième sous-module 430 de mesure disposé sous le récipient 410. Le cinquième sous-module 430 de mesure comprend un ou plusieurs capteurs de poids configuré(s) pour mesurer le poids du récipient 410 lorsque celui-ci est vide ou rempli avec un échantillon, partiellement ou complètement.

Selon un mode de réalisation de l'invention, le troisième module 400 d'analyse comprend des éléments de détection, par exemple des détecteurs optiques ou mécaniques, pour déterminer si un échantillon est présent dans le récipient 410, et de préférence, quel volume de cet échantillon.

Selon un mode de réalisation de l'invention, le troisième module 400 de mesure comprend un élément 415 d'arasement, par exemple une lame d'arasement ou un ressort d'arasement, configuré pour se déplacer au-dessus du récipient 410, ou dans ce récipient 410, afin de mettre à niveau un échantillon contenu dans le récipient 410.

Le déplacement de l'élément 415 d'arasement est automatisé, et garantit une mesure du poids de l'échantillon contenu dans le récipient 410 qui présente une excellente répétabilité.

Selon un mode de réalisation de l'invention (non représenté), l'élément 415 d'arasement comprend un élément de nettoyage et/ou de remise à zéro.

Le cinquième sous-module 430 de mesure est configuré pour transmettre les mesures effectuées à tout moment vers un module de traitement, pour calculer le poids spécifique d'un échantillon quelconque contenu dans le récipient 410 à partir du poids mesuré et du volume mesuré de cet échantillon. Avantageusement, le troisième module 400 d'analyse fournit un moyen compact et précis pour mesurer avec haute précision le poids spécifique d'un échantillon de grains de manière reproductible sur un site extérieur ou à l'intérieur d'un silo à grains.

Le récipient 410 comprend une cinquième sortie 412 pour évacuer et guider l'échantillon hors de celui-ci et hors du troisième module 400 d'analyse, par exemple vers l'extérieur du dispositif 1. La cinquième sortie 412, qui peut être soit en position ouverte ou en position fermée, peut être commandée manuellement ou automatiquement. Selon un mode de réalisation de l'invention, une commande de la cinquième sortie 412 du troisième module 400 d'analyse est réalisée au moyen d'un troisième système 413 de vidange, ce troisième système 413 de vidange comportant par exemple un moteur qui peut être commandé pour ouvrir et fermer la cinquième sortie 412.

Selon un mode de réalisation de l'invention (non représenté), le troisième module 400 d'analyse comprend un système de nettoyage, qui est configuré pour nettoyer la plateforme 416 et/ou l'intérieur du récipient 410 avant ou après une évacuation de l'échantillon hors du récipient 410.

Le troisième module 400 d'analyse permet donc une mesure précise du poids spécifique d'un échantillon contenu dans le récipient 410 peut être obtenue à partir de la mesure du poids de l'échantillon dans le récipient. Le troisième module 400 d'analyse permet en outre une mesure de la tare, c'est-à-dire du poids du récipient lorsque celui-ci est vide. En soustrayant la tare mesurée du poids obtenu en présence de l'échantillon et en utilisant une équation de calibration, il est ainsi possible de déterminer avec précision le poids spécifique de l'échantillon.

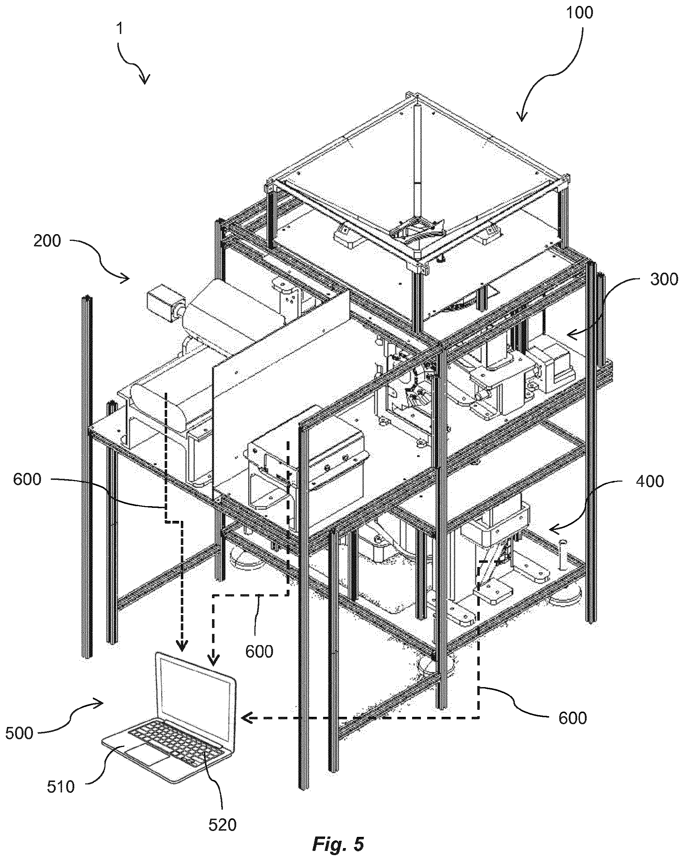

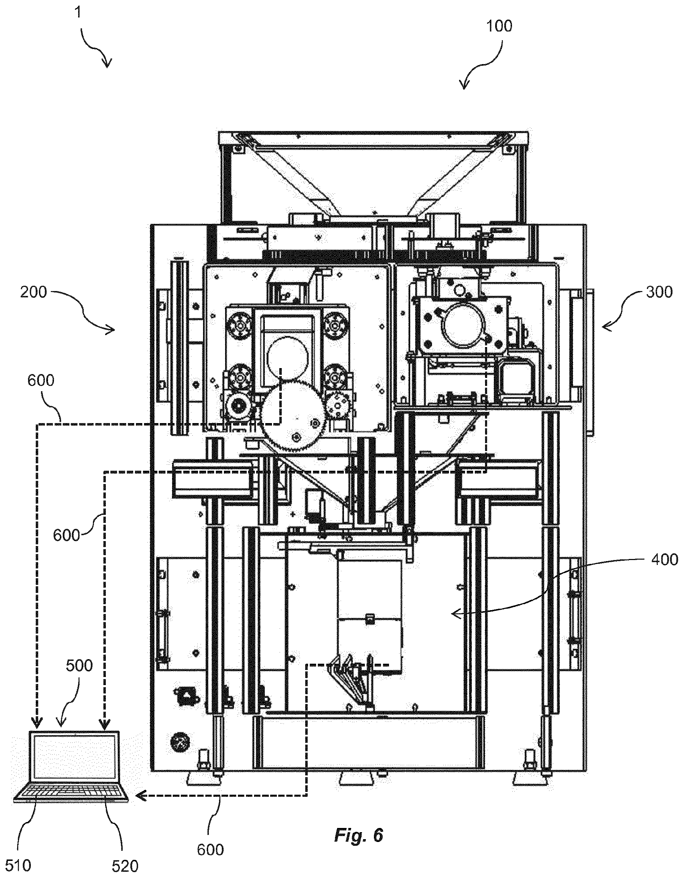

Les figures 5 et 6 montrent, respectivement, une vue en perspective et une vue de côté d'un dispositif 1 selon l'invention, le dispositif 1 comprenant la trémie 100, le premier module 200 d'analyse, le deuxième module 300 d'analyse et le troisième module 400 d'analyse précédemment décrits.

Selon un mode de réalisation de l'invention, la trémie 100 de distribution et le troisième module 400 d'analyse sont chacun reliés au premier module 200 d'analyse et au deuxième module 300 d'analyse au moyen de guides afin de permettre un guidage d'un échantillon de la trémie 100 de distribution vers le premier module 200 d'analyse et vers le deuxième module 300 d'analyse, puis de chacun des premier et deuxième module d'analyse 200 et 300 vers le troisième module 400 d'analyse, sous l'effet de la gravité. L'échantillon est ensuite évacué par le dessous du troisième module 400 d'analyse.

Le dispositif 1 comprend en outre un module 500 de traitement. Sur les figures 5 et 6, le module 500 de traitement est représenté comme un ordinateur portable mais qui peut aussi être tout type de moyen de traitement électronique ou informatique, par exemple un processeur, un ordinateur fixe, un smartphone ou tout appareil similaire à une borne avec un écran de contrôle, une clé USB, une carte mémoire mobile ou tout autre technologie similaire. De préférence, le module 500 de traitement est un PC embarqué.

Le module 500 de traitement est connecté au dispositif 1, en particulier à un ou plusieurs modules d'analyse du dispositif 1, au moyen d'un réseau 600 de communication. Le réseau 600 de communication permet de connecter le module 500 de traitement aux modules 200, 300 et 400 d'analyse. Par exemple, le réseau 600 de communication est un réseau local tel qu'un réseau filaire, un réseau bluetooth, un réseau wifi ou encore un réseau éthernet. Dans tous les cas, le réseau 600 de communication est configuré pour transmettre des informations entre le module 500 de traitement et chaque module d'analyse du dispositif 1.

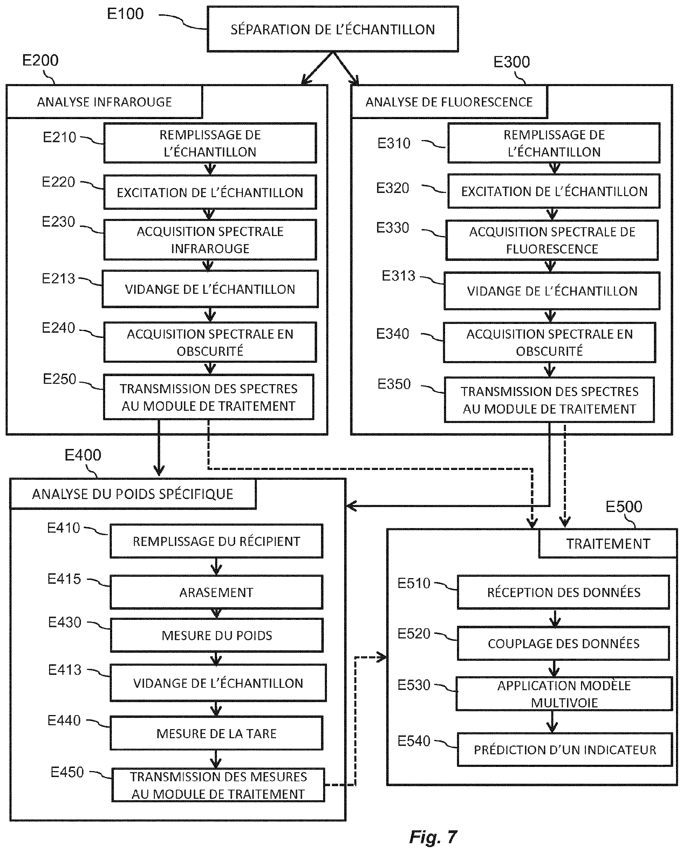

Selon un mode de réalisation de l'invention, le module 500 de traitement est configuré pour contrôler la circulation de l'échantillon dans les différents éléments du dispositif 1, par exemple via la commande d'entrées et de sorties des modules d'analyse 200, 300 et 400 et/ou de la trémie 100. Avantageusement, le module 500 de traitement permet de gérer soit manuellement soit automatiquement les mesures faites par le dispositif 1 en suivant des étapes de procédé telles que décrites par après en rapport avec la figure 7.

Le module 500 de traitement comprend une mémoire 510 qui est configurée pour recevoir des données transmises par le réseau 600 de communication. Ces données peuvent comprendre tout type d'information mesuré par les modules d'analyse tel que des longueurs d'onde des rayonnements émis par l'échantillon dans l'un quelconque des modules d'analyse, des intensités mesurées de ces rayonnements, des spectres électromagnétiques correspondants ou encore des tares et des poids spécifiques mesurés par le troisième module 400 d'analyse

Sur les figures 5 et 6, les flèches discontinues représentent la direction de transmissions des données par le réseau 600 de communication, d'un premier élément vers un deuxième élément. Par exemple, le réseau 600 de communication la transmission d'un spectre infrarouge acquis par le premier module 200 d'analyse vers le module 500 de traitement, d'un spectre de fluorescence acquis par le deuxième module 300 d'analyse vers le module 500 de traitement ou encore d'un poids spécifique mesuré par le troisième module 400 d'analyse vers le module 500 de traitement.

Le module 500 de traitement comprend en outre un processeur 520 qui est configuré pour réaliser des opérations sur les données contenues dans la mémoire 510. Différents logiciels installés sur le processeur 520 peuvent être utilisés pour réaliser ces opérations. En particulier, le processeur 520 est configuré pour réaliser un couplage de spectres, par exemple un couplage d'un spectre infrarouge issu du premier module 200 d'analyse et d'un spectre de fluorescence issu du deuxième module 300 d'analyse, pour produire un spectre mixte. En outre, le processeur 520 du module 500 de traitement est configuré pour déterminer au moins un indicateur de qualité de l'échantillon à partir de ces données ou de ces spectres.

Le processeur 520 est configuré pour organiser les données et les spectres afin d'en réaliser un traitement numérique et informatique, en particulier les spectres de fluorescence, les spectres infrarouge et les poids spécifiques acquis par les modules d'analyse 200, 300 et 400. Par exemple, le processeur 520 organise les données de fluorescence en tableaux mathématiques à trois dimensions, ces trois dimensions correspondant respectivement à la longueur d'onde des rayonnements électromagnétiques d'excitation utilisés, à la longueur d'onde du rayonnement d'émission de l'échantillon mesurée en réponse à cette excitation par un spectromètre, et à l'intensité de ce rayonnement d'émission. Le processeur 520 organise les données infrarouge en tableaux mathématiques distincts, par exemple à deux ou à trois dimensions.

Le processeur 520 est configuré pour réaliser un couplage des données et des spectres, et plus particulièrement des données organisées dans des tableaux mathématiques. Par exemple, une première technique consiste à concaténer les tableaux mathématiques des données de fluorescence et des données infrarouges dans un même tableau mathématique. Avantageusement, cette première technique revient à juxtaposer les spectres correspondants à ces données sur l'intégralité de la gamme des longueurs d'onde des spectres acquis. Une deuxième technique consiste à construire une image spectrale de l'échantillon à partir de la combinaison de spectres de fluorescence et de spectres infrarouges. Avantageusement, cette combinaison permet d'obtenir une image en 3 dimensions qui conserve la structure tridimensionnelle du tableau mathématique des données de fluorescence associées. Une troisième technique consiste à construire une image spectrale bidimensionnelle résultant de la combinaison de la première technique et de la deuxième technique.

Pour réaliser des couplages de données spectroscopiques et déterminer des indicateurs de qualité tels qu'un indice de chute de Hagberg, un taux de contamination en mycotoxines, un taux de contamination en acrylamide, un taux d'humidité, un taux de protéines, une teneur en sucres, une dureté, une force boulangère ou autre caractéristique typique des farines, une granulométrie ou encore un poids spécifique, on pourra se rapporter à l'ouvrage de référence de D. Bertrand et E. Dufour, « La spectroscopie infrarouge et ses applications analytiques» (2006) pour le cas des données issues de spectres infrarouge et à la revue de J. Sadecka et J. Tothova, « Fluorescence Spectroscopy and Chemometrics in the Food Classification - A Review », Czech Journal of Food Sciences 25(4):159-173 (2007) pour le cas des données issues de spectres de fluorescence. Pour la fusion des données, on pourra se reporter aux différentes méthodes évoquées dans la revue « Data fusion methodologies for food and beverage authentication and quality assessment - A review », Analytica Chimica Acta 2015, 891, 1-14.

Une décomposition des données avant ou après fusion est ensuite mise en œuvre par application de modèles statistiques multivariés ou multivoies connus du domaine technique, en vue de prédire un ou plusieurs indicateurs de qualité. Les mesures de poids spécifiques issues du troisième module 400 d'analyse permettent à un utilisateur ou au module 500 de traitement de choisir avec plus de précision les modèles statistiques correspondants au type d'échantillon analysé.

Par ailleurs, le processeur 520 est configuré pour déterminer au moins un indicateur de qualité de l'échantillon à partir des données et des spectres couplés par l'une ou l'autre de ces techniques. Selon un mode de réalisation de l'invention, ces indicateurs de qualités sont choisis parmi : un indice de chute de Hagberg, un taux de contamination en mycotoxines, un taux de contamination en acrylamide, un taux d'humidité, un taux de protéines, une teneur en sucres, une force boulangère ou autre caractéristique des farines, une dureté, une granulométrie ou encore un poids spécifique.

Avantageusement, le calcul d'indicateurs de qualité d'un échantillon à partir de données couplées plutôt que de données obtenues séparément permet d'améliorer la précision de la prédiction d'indicateurs de qualité sélectionnés parmi le taux d'humidité, le taux de protéines, le taux de contamination en mycotoxines, l'indice de chute de Hagberg ou encore le poids spécifique. La connaissance du poids spécifique de l'échantillon mesuré dans le troisième module 400 d'analyse permet d'améliorer d'avantage la précision de cette prédiction, via un choix des modèles statistiques adapté à l'échantillon analysé par le dispositif 1.

Selon un mode de réalisation de l'invention, les dimensions maximales du dispositif 1 sont de 75 centimètres de hauteur, 70 centimètres de largeur et 55 centimètres de profondeur. Par exemple, les dimensions du dispositif 1 sont de l'ordre de 71 centimètres de hauteur, 65 centimètres de largeur et 45 centimètres de profondeur. De préférence les dimensions du dispositif 1 sont de l'ordre de 60 centimètres de hauteur, 55 centimètres de largeur et 50 centimètres de profondeur.

Selon un mode de réalisation de l'invention, le dispositif 1 et/ou certains de ses éléments sont contenus dans un boîtier étanche afin de les isoler des conditions extérieures comme par exemple la présence de poussières, la fluctuation de la température ou encore de chocs liés au transport du dispositif.

La figure 7 montre sous forme d'organigramme des étapes d'un procédé d'analyse d'un échantillon selon un mode de réalisation de l'invention. Selon un mode de réalisation de l'invention, le procédé comprend une première étape E100 de séparation au cours de laquelle un échantillon versé dans la trémie 100 du dispositif 1 est séparé et guidé vers un premier module 200 d'analyse et vers un deuxième module 300 d'analyse. Une deuxième étape E200 d'analyse et une troisième étape E300 d'analyse sont ensuite réalisées, soit successivement soit simultanément.

Selon un mode de réalisation de l'invention, la deuxième étape E200 d'analyse mise en œuvre par le premier module 200 d'analyse vise à acquérir un spectre infrarouge ou proche infrarouge de l'échantillon et comprend les étapes suivantes : une sous-étape E210 de remplissage de la première chambre 210 par un échantillon, une sous-étape E210b de détection du remplissage de la première chambre 210 par les éléments de détection du premier module 200 d'analyse, une sous-étape E220 d'excitation de l'échantillon par le premier sous-module 220 d'excitation, une sous-étape E230 d'acquisition par le premier module 230 d'acquisition d'au moins un spectre infrarouge ou proche infrarouge par le premier module 230 de mesure, une sous-étape E213 d'évacuation de l'échantillon hors de la première chambre 210 et vers le troisième module 400 d'analyse via le premier système 213 de vidange, une sous-étape E240 d'acquisition d'au moins un spectre en obscurité par le premier module 230 lorsque le spectromètre du premier module 230 est obturé par l'obturateur 232 et une sous-étape E250 de transmission des spectres acquis au cours de l'étape E200 vers le module 500 de traitement.

Selon un mode de réalisation de l'invention, une étape de prise de spectre de référence, c'est-à-dire une étape de spectre de la source à vide précède la sous-étape E210 de remplissage. En variante, cette étape de prise de spectre de référence peut suivre directement la sous-étape E213 d'évacuation de l'échantillon.

En variante, la sous-étape E213 d'évacuation de l'échantillon peut être mise en œuvre après l'une ou l'autre parmi la sous-étape E240 d'acquisition d'au moins un spectre en obscurité et la sous-étape E250 de transmission. Selon un autre variante, la sous-étape E240 d'acquisition d'au moins un spectre en obscurité peut être mise en œuvre avant la sous-étape E210 de remplissage de la première chambre 210 par un échantillon. La sous-étape E210 de remplissage peut en outre comporter une sous-étape E210b de détection du remplissage de la première chambre 210 par des éléments de détection du premier module 200 d'analyse.

Selon un mode de réalisation de l'invention, la troisième étape E300 d'analyse mise en œuvre par le deuxième module 300 d'analyse vise à acquérir au moins un spectre de fluorescence de l'échantillon et un spectre en obscurité via les sous-étapes suivantes : une sous-étape E310 de remplissage de la deuxième chambre 310 par un échantillon, une sous-étape E320 d'excitation de l'échantillon par le troisième sous-module 320 d'excitation, une sous-étape E330 d'acquisition par le quatrième sous-module 330 de mesure d'au moins un spectre de fluorescence, une sous-étape E313 d'évacuation de l'échantillon hors de la deuxième chambre 310 via le deuxième système 313 de vidange, une sous-étape E340 d'acquisition d'au moins un spectre en obscurité par le quatrième sous-module 330 de mesure lorsque les sources de lumière du troisième sous-module 320 d'excitation sont éteintes et une sous-étape E350 de transmission des spectres acquis au cours de la troisième étape E300 vers le module 500 de traitement. En variante, la sous-étape E313 d'évacuation de l'échantillon peut être mise en œuvre après l'une ou l'autre parmi la sous-étape E340 d'acquisition d'au moins un spectre en obscurité et la sous-étape E350 de transmission. La sous-étape E310 de remplissage peut en outre comporter une sous-étape E310b de détection du remplissage de la deuxième chambre 310 par des éléments de détection du deuxième module 300 d'analyse.

Selon un mode de réalisation de l'invention, la quatrième étape E400 d'analyse mise en œuvre par le troisième module 400 d'analyse vise à mesurer au moins un poids spécifique de l'échantillon et une tare via les sous-étapes suivantes : une sous-étape E405 de mesure de la tare correspondant au poids à vide du récipient 410, une sous-étape E410 de remplissage du récipient par un échantillon, une sous-étape E415 d'arasement de la surface de l'échantillon par l'élément 415 d'arasement, une sous-étape E420 de mesure du poids spécifique de l'échantillon contenu dans le récipient, une sous-étape E440 d'évacuation de l'échantillon hors du récipient 410 par le troisième système 413 de vidange, et une sous-étape E450 de transmission des mesures effectuées du poids spécifique et de la tare au module 500 de traitement. La sous-étape E410 de remplissage peut comporter en outre une sous-étape E410b de détection du remplissage du récipient 410 par un échantillon, au moyen des éléments de détection du troisième module 400 d'analyse.

Selon un mode de réalisation de l'invention, la cinquième étape E500 de traitement mise en œuvre par le module 500 de traitement vise à déterminer un indicateur de qualité de l'échantillon via les sous-étapes suivantes : une sous-étape E510 de réception des données issues du premier module 200 d'analyse, du deuxième module 300 d'analyse et du troisième module 400 d'analyse, une sous-étape E520 de couplage de ces données, une sous-étape E530 d'application d'au moins un modèle statistique multivoie et une sous-étape E540 de prédiction d'au moins un indicateur de qualité sur la base des sous-étapes précédentes. La cinquième étape E500 de traitement peut en outre inclure une sous-étape E525 non représentée sur la figure 7 consistant à déterminer au moins un modèle statistique multivoie en fonction de la valeur du poids spécifique de l'échantillon transmis au module 500 de traitement au cours de la sous-étape E450, ladite sous-étape E525 étant mise en œuvre avant la sous-étape E530 d'application d'au moins un modèle statistique multivoie.

Naturellement, pour satisfaire des besoins spécifiques, une personne compétente dans le domaine de l'invention pourra appliquer des modifications dans la description précédente. Bien que la présente invention ait été décrite ci-dessus en référence à des modes de réalisation spécifiques, la présente invention n'est pas limitée aux modes de réalisation spécifiques, et les modifications qui se trouvent dans le champ d'application de la présente invention seront évidentes pour une personne versée dans l'art. L'invention est définie par les revendications suivantes.

## Revendications

1. Dispositif (1) d'analyse spectroscopique d'un échantillon de grains, ledit dispositif comprenant un premier module (200)
d'analyse infrarouge, un deuxième module (300) d'analyse de fluorescence, un troisième module (400) d'analyse de poids spécifique, un module (500) de traitement et un réseau de communication (600), chacun desdits premier module (200) et deuxième module (300) comprenant
• une chambre (210, 310) de mesure configurée pour accueillir au moins une partie de l'échantillon ;
• un sous-module (220, 320) d'excitation configuré pour émettre au moins un rayonnement électromagnétique vers ladite au moins une partie de l'échantillon ;
• un sous-module (230, 330) de mesure configuré pour acquérir au moins un spectre électromagnétique de l'échantillon ;
• un système (213, 313) de vidange configuré pour guider l'échantillon vers ledit troisième module (400) ;
le troisième module (400) d'analyse comprenant
• un récipient (410) configuré pour accueillir l'échantillon ;
• un sous-module (430) de mesure configuré pour mesurer un poids spécifique de l'échantillon ;
ledit module (500) de traitement étant connecté à chacun des modules (200, 300, 400) d'analyse par le réseau de communication (600) et comprenant
• une mémoire (510) configurée pour recevoir des données transmises par ledit réseau de communication (600), lesdites données comprenant des spectres électromagnétiques acquis par le premier module (200) et le deuxième module (300) et des poids spécifiques mesurés par le troisième module (400) ; et
• un processeur (520) configuré pour organiser et coupler les données reçues dans la mémoire et pour déterminer un indicateur de qualité de l'échantillon à partir des données couplées.

2. Dispositif selon la revendication 1 dans lequel le sous-module (220) d'excitation du premier module (200) d'analyse infrarouge est configuré pour émettre au moins un rayonnement électromagnétique de longueur d'onde comprise entre 600 et 2500 nanomètres, et dans lequel le sous-module (230) de mesure du premier module (200) d'analyse infrarouge comprend un spectromètre configuré pour acquérir au moins un spectre d'absorbance et/ou de transmittance.

3. Dispositif selon la revendication 1 ou 2, dans lequel le sous-module (320) d'excitation du deuxième module (300) d'analyse de fluorescence est configuré pour émettre au moins un rayonnement électromagnétique de longueur d'onde comprise entre 200 et 800 nanomètres et le sous-module (330) du deuxième module (300) d'analyse de fluorescence comprend un spectromètre configuré pour acquérir au moins un spectre sélectionné parmi : un spectre de fluorescence en mode frontal, acquis à un angle compris entre 30 et 60° par rapport à la surface de l'échantillon, soit un spectre de fluorescence classique, acquis à angle droit, une longueur d'onde dudit au moins un spectre étant comprise entre 200 nanomètres et 800 nanomètres.

4. Dispositif selon l'une quelconque des revendications précédentes, dans lequel ledit dispositif comprend en outre une trémie (100) configurée pour guider au moins une partie de l'échantillon vers le premier module (200) et le deuxième module (300).

5. Dispositif selon l'une quelconque des revendications précédentes, dans lequel ledit indicateur de qualité de l'échantillon est choisi parmi : un indice de chute de Hagberg, un taux de contamination en mycotoxines, un taux de contamination en acrylamides, un taux d'humidité, un taux de protéines, une teneur en sucres, une dureté, une granulométrie ou encore un poids spécifique.

6. Dispositif selon l'une quelconque des revendications précédentes, dans lequel la hauteur du dispositif (1) est inférieure à 75 centimètres, de préférence inférieure à 60 centimètres, la largeur du dispositif (1) est inférieure à 70 centimètres, de préférence inférieure à 55 centimètres, et la profondeur du dispositif (1) est inférieure à 55 centimètres, de préférence inférieure à 50 centimètres.

## Patentansprüche

1. Vorrichtung (1) zur spektroskopischen Analyse einer Kornprobe, wobei die Vorrichtung ein erstes
Infrarotanalysemodul (200), ein zweites Fluoreszenzanalysemodul (300), ein drittes Modul (400) zur Analyse des spezifischen Gewichts, ein Behandlungsmodul (500) und ein Kommunikationsnetz (600) umfasst, wobei jedes des ersten Moduls (200) und des zweiten Moduls (300) umfasst
- eine Messkammer (210, 310), die dazu konfiguriert ist, mindestens einen Teil der Probe aufzunehmen;
- ein Erregungs-Submodul (220, 320), das dazu konfiguriert ist, mindestens eine elektromagnetische Strahlung zu dem mindestens einen Teil der Probe zu emittieren;
- ein Mess-Submodul (230, 330), das dazu konfiguriert ist, mindestens ein elektromagnetisches Spektrum der Probe zu erfassen;
- ein Entleerungssystem (213, 313), das dazu konfiguriert ist, die Probe zu dem dritten Modul (400) zu leiten;
wobei das dritte Analysemodul (400) umfasst
- eine Aufnahme (410), die dazu konfiguriert ist, die Probe aufzunehmen;
- ein Mess-Submodul (430), das dazu konfiguriert ist, ein spezifisches Gewicht der Probe zu messen;
wobei das Behandlungsmodul (500) mit jedem der Analysemodule (200, 300, 400) über das Kommunikationsnetzwerk (600) verbunden ist und umfasst
- einen Speicher (510), der dazu konfiguriert ist, von dem Kommunikationsnetzwerk (600) übertragene Daten zu empfangen, wobei die Daten von dem ersten Modul (200) und dem zweiten Modul (300) erfasste elektromagnetische Spektren und von dem dritten Modul (400) gemessene spezifische Gewichte umfassen; und
- einen Prozessor (520), der dazu konfiguriert ist, die empfangenen Daten im Speicher zu organisieren und zu koppeln und aus den gekoppelten Daten einen Probenqualitätsindikator zu bestimmen.

2. Vorrichtung nach Anspruch 1, wobei das Erregungs-Submodul (220) des ersten Infrarot-Analysemoduls (200) dazu konfiguriert ist, mindestens eine elektromagnetische Strahlung mit einer Wellenlänge zwischen 600 und 2500 Nanometern zu emittieren, und wobei das Mess-Submodul (230) des ersten Infrarot-Analysemoduls (200) ein Spektrometer umfasst, das dazu konfiguriert ist, mindestens ein Absorptions- und/oder Transmissionsspektrum zu erfassen.

3. Vorrichtung nach Anspruch 1 oder 2, wobei das Erregungs-Submodul (320) des zweiten Fluoreszenzanalysemoduls (300) dazu konfiguriert ist, mindestens eine elektromagnetische Strahlung mit einer Wellenlänge zwischen 200 und 800 Nanometern zu emittieren, und das Submodul (330) des zweiten Fluoreszenzanalysemoduls (300) ein Spektrometer umfasst, das dazu konfiguriert ist, mindestens ein Spektrum zu erfassen, das ausgewählt ist aus: einem Frontmodus-Fluoreszenzspektrum, das in einem Winkel zwischen 30 und 60° in Bezug auf die Oberfläche der Probe erfasst wird, oder einem klassischen Fluoreszenzspektrum, das im rechten Winkel erfasst wird, wobei eine Wellenlänge des mindestens einen Spektrums zwischen 200 Nanometern und 800 Nanometern liegt.

4. Vorrichtung nach einem der vorstehenden Ansprüche, wobei die Vorrichtung weiter einen Trichter (100) umfasst, der dazu konfiguriert ist, mindestens einen Teil der Probe zum ersten Modul (200) und zum zweiten Modul (300) zu leiten.

5. Vorrichtung nach einem der vorstehenden Ansprüche, wobei der Probenqualitätsindikator ausgewählt ist aus: einem Hagberg-Fallzahlindex, einem Mykotoxinkontaminationsgrad, einem Acrylamidkontaminationsgrad, einem Feuchtigkeitsgehalt, einem Proteingehalt, einem Zuckergehalt, einer Härte, einer Körnung oder einem spezifischen Gewicht.

6. Vorrichtung nach einem der vorstehenden Ansprüche, wobei die Höhe der Vorrichtung (1) weniger als 75 Zentimeter, vorzugsweise weniger als 60 Zentimeter beträgt, die Breite der Vorrichtung (1) weniger als 70 Zentimeter, vorzugsweise weniger als 55 Zentimeter beträgt und die Tiefe der Vorrichtung (1) weniger als 55 Zentimeter, vorzugsweise weniger als 50 Zentimeter beträgt.

## Claims

1. Device (1) for spectroscopically analysing a grain sample, said device comprising a first infrared analysis module (200), a second fluorescence analysis module (300), a third specific weight analysis module (400), a processing module (500) and a communication network (600), each of said first module (200) and second module (300) comprising
• a measurement chamber (210, 310) configured to receive at least a portion of the sample;
• an excitation submodule (220, 320) configured to emit at least one electromagnetic radiation towards said at least a portion of the sample;
• a measurement submodule (230, 330) configured to acquire at least one electromagnetic spectrum of the sample;
• a draining system (213, 313) configured to guide the sample towards said third module (400);
the third analysis module (400) comprising
• a container (410) configured to receive the sample;
• a measurement sub-module (430) configured to measure a specific weight of the sample;
said processing module (500) being connected to each of the analysis modules (200, 300, 400) by said communication network (600) and comprising
• a memory (510) configured to receive data transmitted by said communication network (600), said data comprising electromagnetic spectra acquired by said first module (200) and said second module (300) and specific weights measured by said third analysis module (400) ; and
• a processor (520) configured to organise and couple the data received in the memory and to determine an indicator of quality of the sample from the coupled data.

2. Device according to claim 1, wherein the excitation submodule (220) of the first infrared analysis module (200) is configured to emit at least one electromagnetic radiation of wavelength comprised between 600 and 2500 nanometres, and wherein the measurement sub-module (230) of the first infrared analysis module (200) comprises a spectrometer configured to acquire at least one absorbance and/or transmittance spectrum.

3. Device according to claim 1 or 2, wherein the excitation submodule (320) of the second fluorescence analysis module (300) is configured to emit at least one electromagnetic radiation of wavelength comprised between 200 and 800 nanometres and the sub-module (330) of the second fluorescence analysis module (300) comprises a spectrometer configured to acquire at least one spectrum selected from among: a fluorescence spectrum in frontal mode, acquired at an angle comprised between 30 and 60° with respect to the surface of the sample, that is a conventional fluorescence spectrum, acquired at a right angle, a wavelength of said at least one spectrum being comprised between 200 nanometres and 800 nanometres.

4. Device according to any one of the preceding claims, wherein said device further comprises a funnel (100) configured to guide at least a portion of the sample towards the first module (200) and the second module (300).

5. Device according to any one of the preceding claims, wherein said indicator of quality of the sample is selected from among: a Hagberg falling number, a mycotoxin contamination rate, an acrylamide contamination rate, a humidity rate, a protein rate, a sugar content, a hardness, a particle size or also a specific weight.

6. Device according to any one of the preceding claims, wherein the height of the device (1) is less than 75 centimetres, preferably less than 60 centimetres, the width of the device (1) is less than 70 centimetres, preferably less than 55 centimetres, and the depth of the device (1) is less than 55 centimetres, preferably less than 50 centimetres.
